# EUROPÄISCHE PATENTSCHRIFT

(11) **EP 3 551 141 B1**
(45) Veröffentlichungstag und Bekanntmachung des Hinweises auf die Patenterteilung: **24.01.2024**
(21) Anmeldenummer: 17822184.2
(22) Anmeldetag: 07.12.2017
(51) Int. Cl.: A61F 5/00

(54) **VORRICHTUNG ZUM EXTERNEN AUFBRINGEN EINER LOKALEN DRUCKKRAFT UNTERHALB DES RIPPENBOGENS EINES PATIENTEN ZUR VERKLEINERUNG DES MAGENVOLUMENS**
DEVICE FOR THE EXTERNAL APPLICATION OF A LOCAL COMPRESSIVE FORCE BELOW THE COSTAL ARCH OF A PATIENT TO REDUCE THE STOMACH VOLUME
DISPOSITIF DESTINÉ À L'APPLICATION EXTERNE D'UNE FORCE DE PRESSION LOCALE EN DESSOUS DE L'ARC COSTAL D'UN PATIENT DESTINÉE À LA RÉDUCTION DU VOLUME DE L'ESTOMAC

(30) Priorität: 08.12.2016 DE 102016015072
(43) Veröffentlichungstag der Anmeldung: 16.10.2019
(73) Patentinhaber: Cebe, Fevzi, 50678 Köln (DE)
(72) Erfinder: Cebe, Fevzi, 50678 Köln (DE)
(74) Vertreter: Thum, Bernhard
(86) Internationale Anmeldenummer: PCT/EP2017/081827
(87) Internationale Veröffentlichungsnummer: WO 2018/104450

(56) Entgegenhaltungen:
- DE-A1- 3 500 078
- DE-A1- 3 500 078
- DE-A1- 19 822 221
- DE-A1- 19 822 221
- US-A- 2 671 899
- US-A- 2 671 899
- US-A- 3 578 773
- US-A- 3 578 773

## Beschreibung

Die vorliegende Erfindung betrifft eine Vorrichtung zum externen Aufbringen einer lokalen Druckkraft unterhalb des Rippenbogens eines Patienten zur Verkleinerung des Magenvolumens.

Die Behandlung von übergewichtigen Personen, die unter Stoffwechselerkrankungen oder Adipositas leiden, gewinnt zunehmend an Bedeutung. Ein Ansatz dieser Behandlung besteht darin, das Magenvolumen eines Patienten zu verkleinern. Hierzu gibt es neben diätischen oder medikamentösen Behandlungsansätzen insbesondere invasive oder chirurgische Behandlungsformen, wie etwa das Anbringen eines Magenbandes, das chirurgische Durchführen einer Magenverkleinerung oder das Einbringen eines Magenballons.

Die chirurgischen Behandlungsformen führen in der Regel kurzfristig und zumindest vorübergehend zu einem gewissen Erfolg, sie lassen sich jedoch bei Patienten, die wiederholt zu Übergewicht neigen, nicht beliebig häufig durchführen, in der Regel nur wenige Male. Eine Magenverkleinerung lässt sich beispielsweise bei einem Patienten in der Regel nur ein einziges Mal vornehmen. Neben diesem Nachteil bringen chirurgische Behandlungsformen die bekannten Nebenwirkungen eines operativen Eingriffs mit sich, sodass diese Behandlungsformen auch nicht für alle Patienten infrage kommen. Darüber hinaus sind chirurgische Eingriffe kostspielig und bergen ein Risiko für den Patienten.

Diätische Behandlungsansätze sind oftmals langwierig und erfordern erhebliche Disziplin von den betroffenen Patienten. Sie führen daher häufig auch nicht zum Erfolg, weil der Patient seinen Lebensstil umstellen muss.

Medikamentöse Behandlungsansätze haben meist erhebliche Nebenwirkungen, die den Organismus des Patienten unnötig belasten.

Es besteht daher ein Bedarf für konservative, d. h. nicht-chirurgische, und auch nicht-medikamentöse Behandlungsansätze für übergewichtige Personen.

Aus dem Stand der Technik sind bereits verschiedene Vorrichtungen bekannt, mit denen versucht wird, auf konservativem Wege das Magenvolumen eines Patienten von außen zu verkleinern.

US 7,264,600 B2 beschreibt einen aktiven Gürtel zur Gewichtskontrolle. Mit diesem lassen sich Luftkissen gezielt aufpumpen. Ferner ist darin eine Art Massagefunktion beschrieben.

DE 102 07 887 A1 offenbart eine Vorrichtung, bei der eine Druckapplizierung pneumatisch oder hydraulisch in der Magengegend von außen erzeugt werden kann.

US 2,671,899 wird als nächstliegender Stand der Technik angesehen und offenbart eine Vorrichtung zum Erzielen eines Druckanstiegs in der Magengegend eines Patienten, wobei ein an einem Gurt angebrachter Hohlkörper vorgesehen ist, der mit einzelnen Belüftungsöffnungen ausgebildet ist und auf die Magengegend Druck ausüben soll.

DE 35 00 078 A1 beschreibt eine Vielzahl von Ausführungsvarianten für einen Bauchgurt zum Applizieren von Druck auf die Magengegend eines Patienten.

WO 2010/042080 A1 beschreibt ein Korsett, das einerseits das äußere Erscheinungsbild eines Patienten durch Formgebung verbessern und andererseits durch gezieltes Ausüben von Druck auf die Magengegend den Appetit eines Patienten zügeln soll. DE 198 22 221 A1 offenbart eine weitere Vorrichtung zum Applizieren von Druck auf die Magengegend eines Patienten mit verschiedenen Polsterelementen.

US 3 578 773 A offenbart einen orthopädischen Gürtel mit einem Drückkörper, wobei gezielt Druck auf bestimmte Muskeln, Verletzungen oder geschwächte Bereiche eines Oberkörpers eines Patienten ausgeübt wird.

Der vorliegenden Erfindung liegt die Aufgabe zugrunde, eine Vorrichtung bereitzustellen, die zur Verkleinerung des Magens eines Patienten geeignet ist.

Diese Aufgabe wird durch eine Vorrichtung zum externen Aufbringen einer lokalen Druckkraft unterhalb des Rippenbogens eines Patienten zur Verkleinerung des Magenvolumens gelöst, wobei die Vorrichtung umfasst:
- einen elastischen Gurt, der um den Oberkörper eines Patienten im Bereich des Rippenbogens unter Zugspannung anlegbar ist, und
- einen Druckkörper, der an dem elastischen Gurt angebracht oder anbringbar ist und der derart ausgestaltet ist, dass er eine Druckkraft von außen auf den Magen im Inneren des Patienten nach Maßgabe der Zugspannung des elastischen Gurts ausübt,
wobei der Druckkörper mit seiner Geometrie der Anatomie des menschlichen Rippenbogens angepasst ist und sich in diesen einschmiegt.

Der Erfinder hat erkannt, dass es grundsätzlich möglich ist, das Magenvolumen eines Patienten durch Aufbringen einer Druckkraft auf den Körper des Patienten im Bereich der Magengegend von außen dauerhaft zu verkleinern und damit in der Folge dessen Hungergefühl zu reduzieren. Um eine solche Druckkraft dauerhaft auf den Körper des Patienten im Bereich der Magengegend auszuüben, wird der unter Zugspannung stehende elastische Gurt um den Oberkörper des Patienten im Bereich des Rippenbogens angelegt. Die durch die elastische Zugspannung des Gurts auf den Oberkörper des Patienten in radialer Richtung ausgeübte Druckkraft wird punktuell bzw. lokal über den Druckkörper auf den Bereich der Magengegend zumindest teilweise konzentriert und so permanent auf den Magenbereich des Patienten eine Druckkraft ausgeübt. Dies wird erfindungsgemäß insbesondere dadurch in vorteilhafter Weise erreicht, dass sich der Druckkörper mit seiner auf den Rippenbogen des Patienten abgestimmten Geometrie in diesen einschmiegt und so die Druckkraft dort appliziert. Mit anderen Worten ist der Druckkörper erfindungsgemäß in Form eines Negativabdrucks des Rippenbogens ausgebildet. In der Folge kommt es - ohne größere Drucckraftverluste am Rippenbogen - durch den Druckkörper zu einer Kompression des Oberbauches im Bereich des Epigastriums (Magens), wodurch die Luft im Magen komprimiert bzw. ausgepresst und dauerhaft das Magenvolumen verkleinert und eingeschränkt wird. In diesem Zusammenhang ist festzuhalten, dass die Organe im Bauchraum in einem luftleeren Raum (Vakuum) liegen und der Magen und der Darm die einzigen Organe sind, die Luft enthalten können. Ein auf den Bauchraum applizierter Druck wird somit unmittelbar auf den Magen und den Darm übertragen und führt an der gewünschten Stelle zu der Volumenreduktion.

Über die Zeit lässt sich mit der erfindungsgemäßen Vorrichtung mittels einer mäßigen aber kontinuierlichen Kompression auf den Magen eine dauerhafte und stetige Volumenreduktion erreichen, welche sich reduzierend auf das Hungergefühl und das Essverhalten des Patienten auswirkt. Dadurch kann mit der erfindungsgemäßen Vorrichtung konservativ, d. h. ohne chirurgischen oder medikamentösen Eingriff, eine Reduzierung des Magenvolumens erreicht werden, sodass der Patient weniger Hunger verspürt und damit über die Zeit aufgrund geringerer Nahrungsaufnahme sein Übergewicht reduzieren wird.

Gemäß einer Ausführungsform der Erfindung kann vorgesehen sein, dass der elastische Gurt geschlossen ist oder in Form eines zumindest teilweise elastischen Bandes ausgeführt ist, das im Bereich seiner Enden über Verbindungsmittel zur Einstellung seiner effektiven Länge um den Oberkörper des Patienten einstellbar miteinander verbindbar ist. Bei einer geschlossenen Ausbildung des Gurts wird dieser vorzugsweise in verschiedenen Größen angeboten, um bei Patienten mit unterschiedlichem Körperumfang einsetzbar zu sein. Vorzugsweise wird aber ein offener, bandförmiger Gurt verwendet, der an seinen Enden mit Verbindungsmitteln verschließbar ist.

In diesem Zusammenhang kann vorgesehen sein, dass die Verbindungsmittel in Form eines Klettverschluss ausgebildet sind, wobei vorzugsweise an einem Ende ein flächiger Klettflauschbereich und an dem anderen Ende ein flächiger Kletthäkchenbereich vorgesehen ist, oder wobei die Verbindungsmittel in Form wenigstens eines verstellbaren Verschlusselementes, wie beispielsweise wenigstens einer Gürtel-Schnallen-Verbindung oder eines Drehrastmeachnismus oder Rastmechanismus oder dergleichen ausgebildet sind. Somit kann der Gurt also beliebig auf Patienten adaptiert werden, wobei vorzugsweise verschiedene Gurtlängen als Grundgrößen angeboten werden.

Eine Weiterbildung der Erfindung sieht vor, dass der Druckkörper in Form eines flächigen flexiblen Druckkörpers ausgebildet ist. Dabei kann der Druckkörper insbesondere in Form eines Gummi-, Kunststoff- oder Silikonkörpers ausgebildet sein. Maßgeblich ist, wie eingangs bereits ausgeführt, dass der Druckkörper derart gestaltet ist, dass er zum Angreifen unterhalb des Rippenbogens geeignet ist, d. h. derart, dass der Druckkörper, mit dem die Druckkraft über die Haut des Patienten auf die Magengegend ausgeübt wird, unterhalb des Rippenbogens in den Magenbereich eingreifen kann. Dadurch lässt sich gezielt die gewünschte Druckkraft auf die Magengegend ausüben und so dauerhaft das Magenvolumen konservativ reduzieren.

Eine beispielhafte Weiterbildung sieht in diesem Zusammenhang vor, dass der Druckkörper in Form eines abgerundeten Vielecks, insbesondere eines abgerundeten Dreiecks, einer Ellipse oder eines Ovals ausgebildet ist. Je nach Anatomie, Alter und persönlichem Befinden lässt sich eine geeignete Geometrie für den Druckkörper in Abhängigkeit vom Patienten auswählen.

Gemäß einer vorteilhaften Weiterbildung der Erfindung ist der Druckkörper mit einem umgekehrt V-förmigen Erhebungsbereich ausgebildet ist, der im Bereich des V-Scheitels seine größte Materialstärke aufweist. Der V-förmige Erhebungsbereich mit seiner größten Erhebung im Bereich des V-Scheitels fügt sich anatomisch in den Rippenbogen ein und sorgt an seinem Bereich der größten Erhebung für die maximale Druckapplizierung (Epigastrium), idealerweise am oder im Bereich des Solar Plexus des Patienten. Der Erhebungsbereich entlang der V-Schenkel erhöht die Druckkonzentration im Oberbauch.

Um ein Verrutschen des Druckkörpers am Patienten zu vermeiden und um den Tragekomfort zu erhöhen, kann gemäß einer Weiterbildung der Erfindung vorgesehen sein, dass der Druckkörper an seiner am Patienten angreifenden Oberfläche mit vorspringenden Noppen versehen ist. Durch diese Maßnahme bleibt der Druckkörper besser in seiner Sollposition am Patienten. Darüber hinaus wird auch bei einem dauerhaften Tragen der erfindungsgemäßen Vorrichtung eine gewisse Luftzirkulation im Bereich des Druckkörpers möglich sein, was den Tragekomfort erhöht.

Ferner kann gemäß einer Weiterbildung der Erfindung vorgesehen sein, dass an der Rückseite des Druckkörpers Befestigungsmittel vorgesehen sind, insbesondere ein Klettflauschbereich oder ein Kletthäkchenbereich. Damit lässt sich der Druckkörper am Gurt oder, wie im Folgenden näher beschrieben, an einem zwischen dem Gurt und dem Druckkörper zusätzlich angeordnetem Druckkissen fixieren. Alternativ zu einem Klettverschluss können auch Befestigungsmittel, wie Knöpfe, Druckknöpfe Rastelemente, Formschlusselemente, Magnete oder dergleichen vorgesehen sein.

Eine Weiterbildung der Erfindung sieht vor, dass zwischen dem Druckkörper und einer zur Anlage an dem Patienten vorgesehenen Innenseite des Gurts ein Druckkissen vorgesehen ist. Eine solche zusätzliche Anordnung eines Druckkissens, im Fachjargon auch "Pelotte" genannt, ermöglicht bei geeigneter Auswahl der Geometrie dieses Druckkissens eine auf die Anatomie des Patienten abgestimmte punktuelle oder flächige Applizierung der Druckkraft.

Aus Gründen des Tragekomforts kann der Druckkörper nicht beliebig steif ausgebildet werden. Vielmehr ist bei der Gestaltung des Druckkörpers, der unmittelbar in Kontakt mit der Haut des Patienten tritt, auf Hautverträglichkeit und die Vermeidung von unangenehmen Druckstellen oder Reibestellen zu achten, die zu Hautirritationen führen können. Vorzugsweise ist der Druckkörper mit einer hautfreundlichen Beschichtung oder einem Überzug, insbesondere aus einem reinigungsfähigen oder hygienischen Textilmaterial versehen.

Das Druckkissen kann jedoch, weil es nicht in unmittelbaren Kontakt mit der Haut des Patienten kommt, aus einem härteren Material, beispielsweise aus einem formstabilen, vorzugsweise elastischen Material, vorzugsweise aus Gummi, Kunststoff oder Silikon ausgebildet werden. Auch das Druckkissen kann mit einer Beschichtung oder einem reinigbaren Überzug versehen sein. Allerdings kann das Druckkissen mit seiner Geometrie insbesondere im Hinblick auf den gewünschten Sollbereich geometrisch abgestimmt werden, in dem die Druckkraft auf den Patienten zur Verkleinerung des Magenvolumens ausgeübt werden soll. Ferner kann vorgesehen sein, dass der Druckkörper mit einem Flauschmaterial überzogen ist, wobei das Flauschmaterial unmittelbar an dem Druckkörper angeformt ist oder in Form einer den Druckkörper aufnehmenden auswechselbaren Stofftasche ausgebildet ist.

Hinsichtlich der Geometrie des Druckkissens kann vorgesehen sein, dass dieses zumindest einseitig eine konvexe, d. h. nach außen gewölbte, Form aufweist, wobei das Druckkissen insbesondere im Querschnitt rechteckig, abgerundet rechteckig, elliptisch oder oval ausgebildet sein kann. Wie vorstehend angedeutet, können je nach den anatomischen Gegebenheiten des Patienten und der Behandlungssituation Druckkissen unterschiedlicher Geometrie und Härte verwendet werden.

Zur Fixierung des Druckkissens zwischen dem Gurt und dem Druckkörper sieht eine Weiterbildung der Erfindung vor, dass das Druckkissen an seiner dem Gurt zugewandten Seite sowie an seiner dem Druckkörper zugewandten Seite jeweils mit Befestigungsmitteln, insbesondere mit einem Klettflauschbereich oder ein Kletthäkchenbereich ausgebildet ist. Alternativ zu einer Befestigung über einen Klettverschluss lassen sich zur Fixierung des Druckkissens alternativ Knöpfe, Druckknöpfe, Rastelemente, Formschlusselemente, Magnete oder dergleichen einsetzen.

Eine Weiterbildung der Erfindung sieht vor, dass der Gurt in einem Bereich, in dem der Druckkörper, gegebenenfalls unter Vermittlung des vorstehend beschriebenen Druckkissens, anbringbar ist, durch ein formstabiles Verstärkungselement verstärkt ist. Durch ein solches Verstärkungselement kann beispielsweise das Druckkissen und oder der Druckkörper in seiner Lage relativ zum Patienten stabilisiert werden, wodurch das dauerhafte Applizieren einer Druckkraft noch zuverlässiger gewährleistet ist. In diesem Zusammenhang kann vorgesehen sein, dass der Bereich, in dem der Druckkörper, gegebenenfalls unter Vermittlung des Druckkissens, anbringbar ist, in einem mittleren Abschnitt oder in einem Endbereich des Gurts angeordnet ist. Je nach Ausgestaltung kann dies dazu führen, dass sich der Gurt beispielsweise in einem Rückenbereich des Patienten oder im Brustbereich des Patienten verschließen lässt.

Beispielsweise kann in einer Ausführungsvariante der Erfindung das Verstärkungselement einen gerundet ausgebildeten Scheitelbereich aufweisen, der außermittig, vorzugsweise in einem Bereich eines Drittels der Gesamtlänge des Verstärkungselements, angeordnet ist, wobei vorzugsweise das Verstärkungselement in seinem Scheitelbereich einen Öffnungswinkel von 170° bis 120°, am meisten bevorzugt einen Öffnungswinkel von etwa 150° aufweist. Dadurch ist es möglich, den Scheitelbereich in Abstimmung mit dem Druckkörper zur maximalen Druckkraftapplizierung in Ausrichtung mit dem Bereich der maximalen Materialstärke bzw. maximalen Erhebung des Körpers zu bringen.

Diese Ausgestaltung des Verstärkungselements, erlaubt aber auch eine variable Nutzung des Verstärkungselements in unterschiedlichen Anordnungen relativ zum Druckkörper, um so je nach Bedarf des Patienten weniger oder mehr Druck auf den Magenbereich zu applizieren. Dabei kann vorgesehen sein, dass das Verstärkungselement wahlweise in konvexer oder konkaver Anordnung relativ zu dem Druckkörper oder/und wahlweise mit seinem Scheitelbereich nahe oder entfernt von einer maximalen Materialstärke des Druckkörpers anordenbar ist. Mit anderen Worten ist es möglich, je nach Abhängigkeit der Ausrichtung des Verstärkungselements die vom Druckkörper auf den Magenbereich applizierte Druckkraft über das Verstärkungselement mehr oder weniger stark zu unterstützen.

Vorzugsweise sieht eine Weiterbildung der Erfindung vor, dass der Gurt eine Ausnehmung oder Aufnahmetasche zum Aufnehmen des Verstärkungselements aufweist. Dadurch ist gewährleistet, dass das Verstärkungselement im Gurt lagestabil bleibt.

Ferner sieht eine Weiterbildung der Erfindung vor, dass das Verstärkungselement von einer bogenförmig gekrümmten Verstärkungsleiste gebildet ist, die mit zwei gegenüber einem Mittelbereich vorspringenden Endbereichen versehen ist, wobei das Verstärkungselement derart in dem Gurt aufgenommen oder an diesem angebracht ist, dass die beiden Endbereiche in Richtung zum Patienten hin vorstehen. Die Verstärkungsleiste kann beispielsweise eine Metallleiste oder eine stabile Kunststoffleiste aus einem thermalplastischen Material oder dergleichen sein.

Eine Weiterbildung der Erfindung sieht vor, dass in dem Gurt in einem mit der Haut des Patienten in Kontakt stehenden Bereich, insbesondere in dem Druckkörper, wenigstens ein Sensor zur Erfassung von Patientenparametern vorgesehen ist, insbesondere ein Sensor zu Erfassen von Puls oder/und Herzfrequenz oder/und Blutdruck oder/und Blutzucker oder/und Atemfrequenz oder/und Körpertemperatur oder/und Fettanteil oder/und Wasseranteil oder/und Muskelanteil oder/und Body-Mass-Index oder dergleichen. Derartige Sensoren können Elektroden umfassen, die in unmittelbaren Kontakt mit der Haut des Patienten gelangen. Diese Sensoren lassen sich beispielsweise von einer gemeinsamen Versorgungseinheit in Form einer Batterie versorgen. Die Sensoren lassen sich über eine kabelgebundene Schnittstelle oder kabellos beispielsweise über IR, Bluetooth, WI-FI oder eine Mobilfunkverbindung, mit einer Auswerteeinheit, beispielsweise einem Smartphone oder einer Smartwatch oder einem Computer oder einem Tablet oder einer elektronischen Waage oder einer sonstigen elektronischen Auswerteeinheit koppeln. Auch eine Fernüberwachung von Patientendaten und Patientenparametern direkt über eine in oder an dem Gurt angeordnete Mobilfunkschnittstelle oder unter Zwischenschaltung eines Sendegeräts, wie beispielsweise einem Smartphone oder einer Smartwatch ist erfindungsgemäß möglich.

Im Folgenden werden verschiedene Ausführungsformen der Erfindung anhand der beiliegenden Figuren beispielhaft erläutert. Es stellen dar:
- Fig. 1: eine schematische perspektivische Ansicht einer erfindungsgemäßen Vorrichtung gemäß einer ersten Ausführungsform;
- Fig. 2: eine vergrößerte Vorderansicht des dreieckförmigen Druckkörpers gemäß der ersten Ausführungsform der Erfindung;
- Fig. 3: eine Draufsicht auf den dreieckförmigen Druckkörper mit elliptischem Druckkissen gemäß der ersten Ausführungsform der Erfindung;
- Fig. 4: eine Rückansicht des dreieckförmigen Druckkörpers gemäß der ersten Ausführungsform der Erfindung;
- Fig. 5: eine Rückansicht des Druckkissens gemäß der ersten Ausführungsform der Erfindung;
- Fig. 6: eine Vorderansicht des Druckkissens gemäß der ersten Ausführungsform der Erfindung;
- Fig. 7a - 7c: verschiedene Draufsichten auf Druckkissen zur Erläuterung unterschiedlicher Geometrien für Druckkissen gemäß verschiedener Ausführungsformen der Erfindung;
- Fig. 8: eine perspektivische Ansicht eines gekrümmten Verstärkungselements gemäß der ersten Ausführungsform der Erfindung;
- Fig. 9: eine schematische Darstellung einer Vorrichtung gemäß einer zweiten Ausführungsform der vorliegenden Erfindung;
- Fig. 10: eine schematische Darstellung eines Patienten an dem eine Vorrichtung gemäß der vorliegenden Erfindung zur Behandlung angebracht ist;
- Fig. 11: eine alternative Ausgestaltung eines Verstärkungselements gemäß einer weiteren Ausführungsform der Erfindung zeigt;
- Fig. 12: eine alternative Ausgestaltung eines Druckkörpers gemäß einer weiteren Ausführungsform der Erfindung zeigt; und
- Fig. 13a bis 13e: verschiedene Kombinationsmöglichkeiten des Druckkörpers gemäß Figur 12 mit dem Verstärkungselement gemäß Figur 11 zeigen.

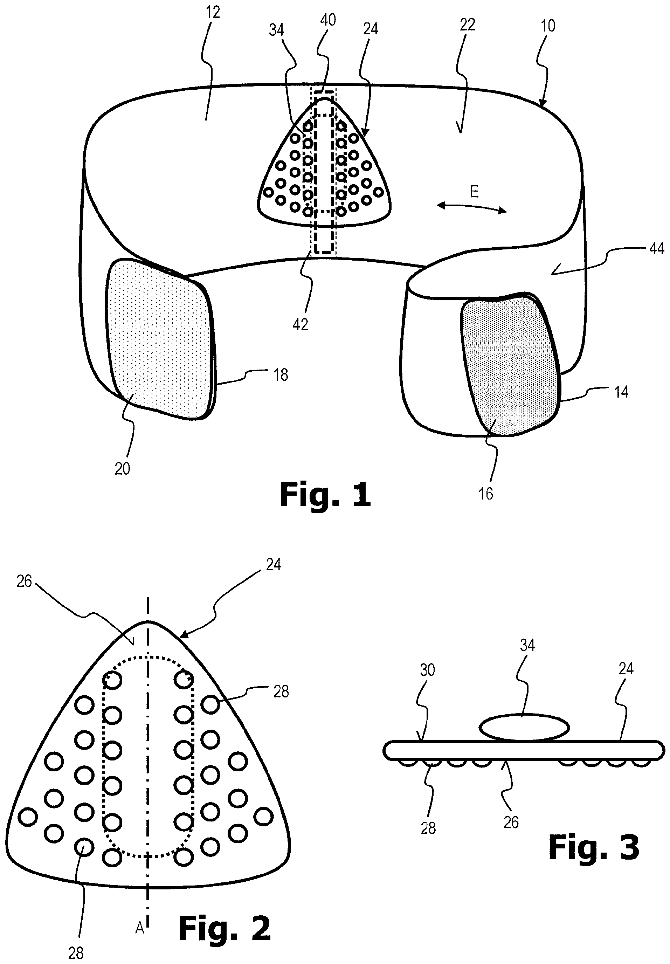

In Figur 1 ist eine Vorrichtung gemäß der vorliegenden Erfindung in perspektivischer Darstellung gezeigt und allgemein mit 10 bezeichnet. Die Vorrichtung 10 umfasst einen Gurt 12 aus einem elastischen Material, das entlang seiner Längsrichtung gemäß dem Pfeil E elastisch dehnbar ist. Der Gurt 12 weist an seinem einen Ende 14 ein flächiges Klettverschlussfeld 16 auf, das mit Kletthäckchen versehen ist. Ferner weist der Gurt 12 an seinem anderen Ende 18 ein flächiges Klettverschlussfeld 20 auf, das als Klettflausch ausgebildet ist, wobei zum Verschließen des Gurts 12 um einen Oberkörper eines Patienten herum die Kletthäkchen des Feldes 16 mit dem Klettflausch des Feldes 20 variabel hinreichend fest in Eingriff bringbar sind, um auch unter elastischen Zugspannung zusammen zu halten.

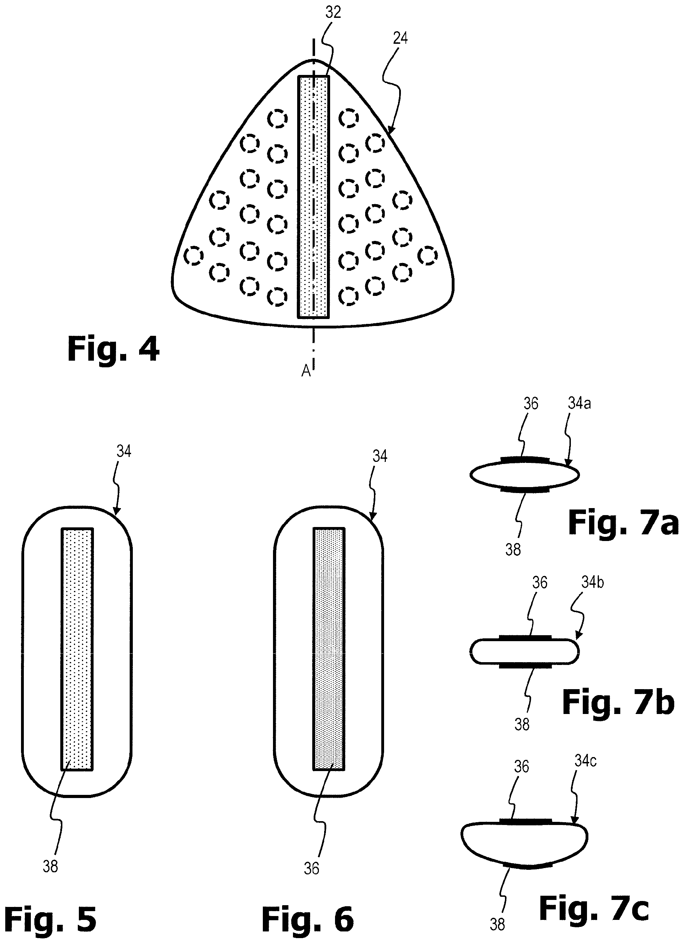

In seinem mittleren Bereich weist der Gurt 12 an seiner Innenseite 22 einen Drucckörper 24 auf, der in Form eines Dreiecks mit abgerundeten Ecken ausgebildet ist. der Druckkörper 24 ist in Vorderansicht in Figur 2, in Draufsicht in Figur 3 und in Rückansicht in Figur 4 dargestellt. Der Druckkörper 24 ist aus einem formstabilen, jedoch deformierbaren Gummimaterial hergestellt und zumindest an seiner Vorderseite 26 mit einem hygienischen Überzugmaterial überzogen, das einfach zu reinigen ist. Ferner weist der Druckkörper 24 an seiner Vorderseite 26 eine Reihe von gerundet ausgebildeten noppenartigen Vorsprüngen 28 auf, die insbesondere seitlich einer gedachten Mittelachse A in regelmäßigen Mustern angeordnet sind. In dem Bereich um die Mittelachse A herum sind keine derartigen Vorsprünge 28 vorgesehen, weil dort der größte Anteil der Druckkraft ausgeübt wird.

Wie in Figur 4 gezeigt, ist an der Rückseite 30 des Druckkörpers 24 im Bereich seiner Mittelachse A ein Streifen 32 aus einem Material mit Kletthäkchen fest angebracht.

Ferner erkennt man in Figur 3, dass an der Rückseite 30 des Druckkörpers 24 ein Druckkissen 34 vorgesehen ist. Zur Erläuterung des Druckkissens wird auf die Figuren 5, 6 und 7a bis 7c verwiesen. Figur 5 zeigt eine Rückansicht des Druckkissens 34, wohingegen Figur 6 eine Vorderansicht des Druckkissens 34 zeigt. Figuren 7a bis 7c zeigen verschiedene Geometrien, die das Druckkissen in der Draufsicht aufweist. Das Druckkissen 34 ist als formstabiler und relativ harter länglicher Silikonkörper mit abgerundeten Ecken und abgeflachten Randbereichen ausgebildet. An seiner Vorderseite (Figur 6) weist es einen Bereich 36 auf, der mit in einem Klettflausch versehen und etwa so dimensioniert ist, wie der Bereich 32 des Druckkörpers. Zur Anbringung des Druckkörpers 24 an dem Druckkissen 34 werden die Bereiche 32 und 36 als Klettverschluss miteinander in Eingriff gebracht. An seiner Rückseite weist das Druckkissen 34 gemäß Figur 5 wiederum ein Klettverschlussfeld 38 auf, das mit Kletthäkchen versehen ist.

Figuren 7a bis 7c zeigen verschiedene Geometrien, die das Druckkissen aufweisen kann. In Figur 7b weist das Druckkissen 34a in der Draufsicht eine elliptische Form auf, wobei die beiden Klettverschlussfelder 36 und 38 auf der Vorderseite und auf der Rückseite angeordnet sind. In der Ausführungsform gemäß Figur 7b weist das Druckkissen 34b eine gerundete rechteckige Form oder ovale Form auf, wobei wiederum die beiden Klettverschlussfelder 36 und 38 auf der Vorderseite und auf der Rückseite angeordnet sind. In der Ausführungsform gemäß Figur 7c weist das Drucckissen 34c auf der Vorderseite eine stark konvex gewölbte Form mit dem Klettverschlussfeld 36 auf, wohingegen es auf seiner Rückseite mit dem Klettverschlussfeld 38 eine flache Form aufweist. Das Klettverschlussfeld 38 ist dafür vorgesehen, um das Druckkissen an einem korrespondierenden Klettverschlussfeld mit einem Klettflausch an der Innenseite 22 des Gurts 12 oder direkt ohne Vermittlung eines weiteren Klettverschlussfeld direkt an der Innenseite 22 des Gurts 12 fixiert zu werden.

Es versteht sich, dass beim Gegenstand der vorliegenden Erfindung unabhängig vom jeweiligen Ausführungsbeispiel das Druckkissen auch andere Geometrien aufweisen kann. Beispielsweise kann es kürzer gestaltet werden, bauchiger, stärker konvex oder weniger konvex gekrümmt, in Richtung zum Patienten hin abgeflacht oder stärker gekrümmt, in Richtung zur Rückseite, d. h. zum Gurt hin, abgeflacht oder stärker gekrümmt, in der Vorder- und Rückansicht bauchiger, kreisrund, elliptisch oder ebenfalls dreieckförmig.

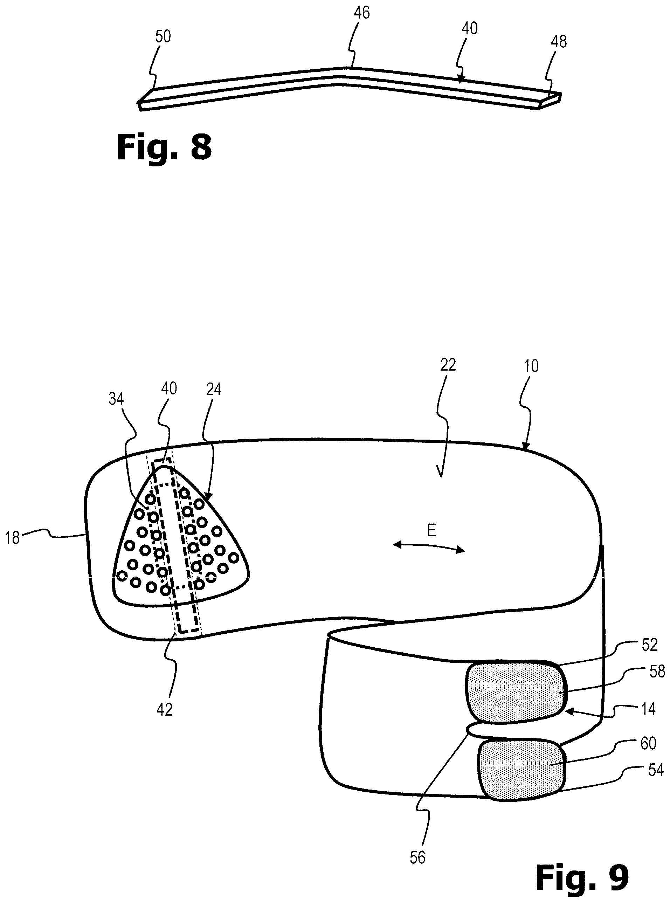

Figur 8 zeigt ein Verstärkungselement 40. Dieses Verstärkungselement 40 ist dazu vorgesehen, in einer Aufnahmetasche 42 am Gurt 12 (siehe Figur 1) angebracht zu werden. Das Verstärkungselement 40 ist aus einem formstabilen Material, beispielsweise aus Metall oder einem harten Kunststoffmaterial, wie etwa einem Thermoplast, hergestellt. Die Aufnahmetasche 42 ist entweder auf der Innenseite 22 des Gurts 12 oder auf der Außenseite 44 des Gurts 12 angebracht. Sie ist in Figur 1 lediglich durch Strichlinien gezeigt, weil sie bei der Ausführungsform gemäß Figur 1 auf der Außenseite 44 angebracht ist. Das Verstärkungselement 40 ist gekrümmt mit einem Winkel von ca. 15° ausgebildet. Der Winkel kann je nach Bedarf größer oder kleiner gewählt werden. Durch die Krümmung bildet sich im mittleren Bereich des Verstärkungselements 40 ein Scheitel 46 aus, der etwa im mittleren Bereich des Gurts 12 liegt. Dabei ist die Orientierung des Verstärkungselements 40 derart vorgesehen, dass dieses in seinen Endbereichen 48, 50 zum Patienten hin gekrümmt ist, wobei der Scheitel 46, vom Patienten weg weist. Durch diese Anordnung kann der Gurt im Bereich des Druckkissens 34 mit dem Verstärkungselement 40 stabilisiert werden, um so über das Druckkissen 34 und schließlich den Druckkörper 24 besser Druck auf den Magenbereich des Patienten auszuüben.

Figur 9 zeigt eine zweite Ausführungsform der Erfindung, die im Wesentlichen mit der ersten Ausführungsform übereinstimmt, sich jedoch in folgenden Aspekten vom Gegenstand der ersten Ausführungsform unterscheidet:
- Der Druckkörper 24, das Druckkissen 34, die Aufnahmetasche 42 und das Verstärkungselement 40 sind nahe dem Ende 18 des Gurts 12 angeordnet.
- Das andere Ende 14 weist zwei Laschen 52 und 54 auf, die über einen Schlitz 56 voneinander getrennt sind.
- Jede dieser Laschen 52, 54 weist ein jeweiliges Klettverschlussfeld 58, 60 mit Kletthäkchen auf.

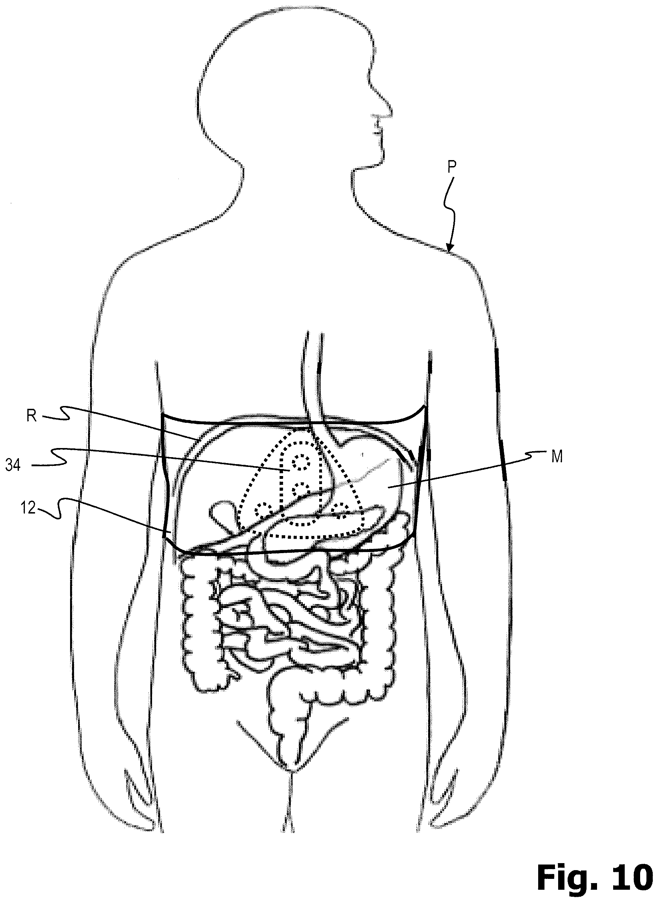

Figur 10 zeigt zur Veranschaulichung der Anwendung der Vorrichtung gemäß der vorliegenden Erfindung einen Patienten P in schematischer Darstellung, wobei dessen Organe des Verdauungstraktes im Einzelnen schematisch gezeigt sind. Insbesondere erkennt man die Darstellung des Magens M, der bei dem Patienten etwas größer ausgebildet gezeigt ist. Zur Reduzierung des Magenvolumens ist an dem Patienten der Gurt 12 gemäß der vorliegenden Erfindung angelegt, wobei über den Druckkörper 24 und das Druckkissen 36 eine Druckkraft unterhalb des Rippenbogens R ausgeübt wird, um das für den Magen zur M Verfügung stehende Volumen zu verkleinern. Dadurch lässt sich wirksam dauerhaft das Hungergefühl des Patienten reduzieren und wirksam Übergewicht bekämpfen.

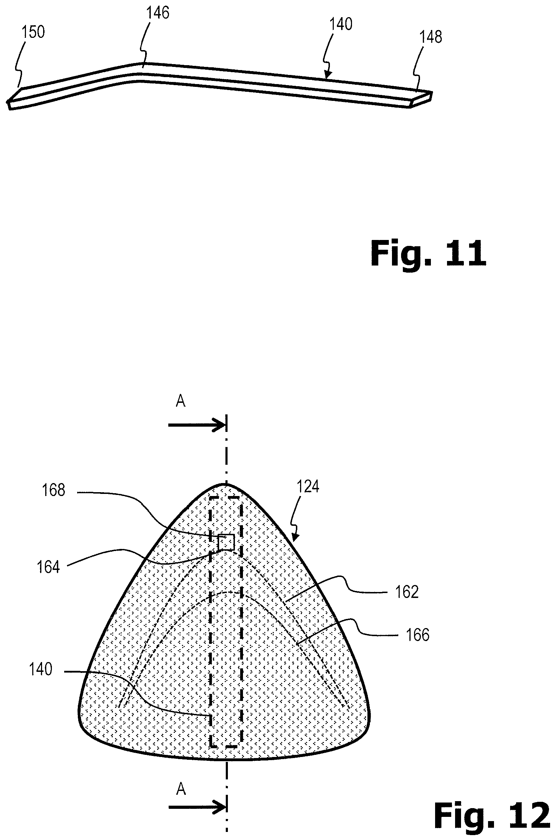

Figur 11 zeigt eine alternative Ausführungsform für ein Verstärkungselement 140, wobei der Scheitel 146 in einem Bereich an der Grenze zwischen dem ersten und dem zweite Drittel der Gesamtlänge des Verstärkungselements liegt. Der Öffnungswinkel liegt in einem Bereich von 150°.

Figur 12 zeigt eine alternative Ausführungsform für einen weiteren Druckkörper 124. Diese ist beispielsweise aus einem formstabilen Gummimaterial hergestellt und weist eine dreieckförmige Geometrie auf, wobei diese der Kontur des menschlichen Rippenbogens nachempfunden ist. Der Druckkörper 124 ist mit einem Flauschmaterial beschichtet. Die Konturlinie 162 kann als Höhenlinie verstanden werden und bildet sozusagen einen Grat einer V-förmigen Erhebung, die an ihrem Scheitelpunkt 164 die maximale Erhebung erreicht. Ferner zeigt eine zweite Konturlinie 166 den Bereich einer deutlich reduzierten Materialstärke des Druckkörpers 124 unterhalb der Konturlinie 162.

An dem Scheitelpunkt 164 ist ein Sensor 168 vorgesehen, der zur Erhebung von Patientendaten dient, beispielsweise von Puls oder/und Herzfrequenz oder/und Blutdruck oder/und Blutzucker oder/und Atemfrequenz oder/und Körpertemperatur oder/und Fettanteil oder/und Wasseranteil oder/und Muskelanteil oder/und Body-Mass-Index oder dergleichen. Dieser Sensor 168 ist über eine kabellose Schnittstelle mit einem Nutzer-Endgerät, beispielsweise einem Smartphone, zur Datenübertragung koppelbar. Ferner erkennt man in Figur 12 angedeutet das Verstärkungselement 140.

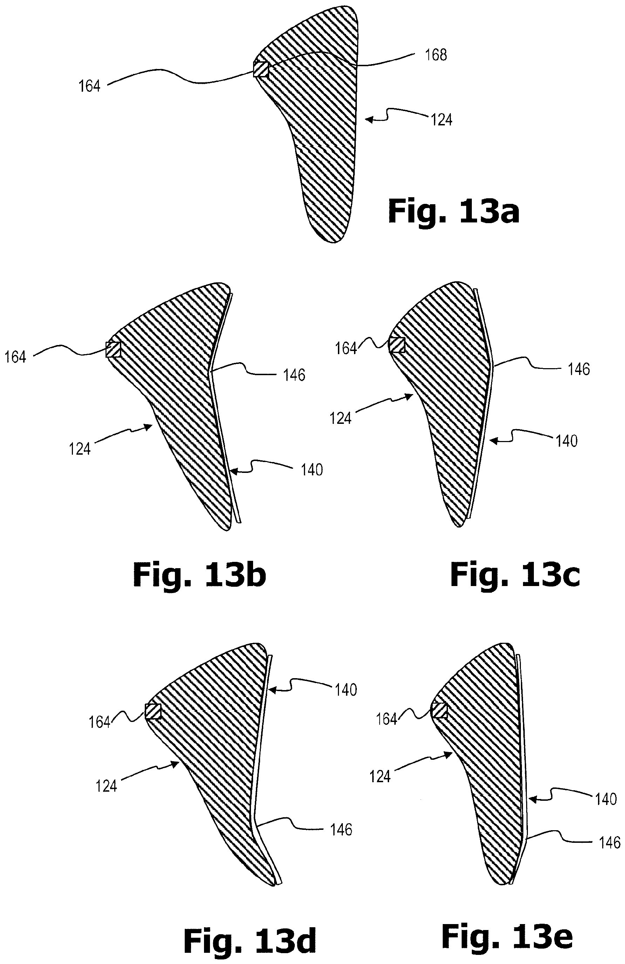

Figur 13a zeigt einen Schnitt durch den Druckkörper 124 entlang der Schnittlinie A-A. Man erkennt die massive Ausgestaltung aus einem deformierbaren Schaumstoff oder Gummimaterial, das hinreichend formstabil ist, um Druck auf die Magengegend eines Patienten auszuüben. Ferner erkennt man den Bereich 164 maximaler Erhebung sowie den Sensor 168. In der Darstellung gemäß Figur 13a ist der Druckkörper 124 ohne Verstärkungselement gezeigt.

Figuren 13b bis 13e zeigen verschiedene Möglichkeiten zur Anordnung des Verstärkungselements 140 am Druckkörper 124.

Bei der Anordnung gemäß Figur 13b ist das Verstärkungselement 140 mit seinem Scheitelbereich 146 derart angeordnet, dass der Scheitel 146 konvex in das Verstärkungselement 140 im Bereich der maximalen Erhebung 164 hinein drückt und so für eine maximale Unterstützung des Druckkörpers 124 zur Druckkraftapplizierung auf die Magengegend des Patienten sorgt.

Bei der Anordnung gemäß Figur 13c ist das Verstärkungselement 140 mit seinem Scheitelbereich 146 derart angeordnet, dass der Scheitel 146 in etwa auf der Höhe der maximalen Erhebung 164 des Druckkörpers 124 liegt, jedoch zu diesem konkav angeordnet ist. Demzufolge kann das Material des Druckkörpers 124 sozusagen in den konkaven Scheitelbereich 146 ausweichen. Dadurch wird die Druckkraftapplizierung auf die Magengegend eines Patienten deutlich geringer als bei der Anordnung gemäß Figur 13b.

Bei der Anordnung gemäß Figur 13c ist das Verstärkungselement 140 wieder in konvexer Anordnung am Druckkörper 124 vorgesehen, wobei jedoch der Scheitelbereich 146 deutlich unterhalb des Bereichs der maximalen Erhebung 164 liegt. Dadurch wird eine gegenüber dem Zustand gemäß Figur 13b reduzierte Applizierung von Drucckraft auf die Magengegend des Patienten erreicht.

Bei der Anordnung gemäß Figur 13e ist das Verstärkungselement 140 gegenüber dem Zustand aus Figur 13d um 180° gedreht, sodass sich wiederum eine konkave Anordnung des Scheitelbereichs 146 ergibt. Dadurch ist die Applizierung von Drucckraft auf die Magengegend des Patienten weiter reduziert, weil Material des Drucckörpers 124 in den Scheitelbereich 146 ausweichen kann.

Insgesamt ergeben sich durch die unterschiedlichen Möglichkeiten zur Anordnung des Druckkörpers 124 und des Verstärkungselements 140 verschiedene Variationsmöglichkeiten zur Einstellung der auf die Magengegend des Patienten ausgeübten Druckkraft. So lässt sich beispielsweise im Rahmen einer Therapie durch veränderte Anordnung des Verstärkungselements 140 zunächst weniger und später mehr Druck auf die Magengegend ausüben, um so den Therapieverlauf zu steuern, beispielsweise mit einem schonenden Therapiebeginn unter Anwendung einer geringen Druckkraft auf die Magengegend mit anschließend schrittweise Steigerung der Druckkraftapplizierung.

## Patentansprüche

1. Vorrichtung (10) zum externen Aufbringen einer lokalen Druckkraft unterhalb des Rippenbogens (R) eines Patienten zur Verkleinerung des Magenvolumens, umfassend
- einen Gurt (12), der um den Oberkörper eines Patienten im Bereich des Rippenbogens (R) unter Zugspannung anlegbar ist, und
- einen Druckkörper (24), der an dem Gurt (12) anbringbar ist und der derart ausgestaltet ist, dass er, in Gebrauch, eine Druckkraft auf den Magen im Inneren des Patienten nach Maßgabe der Zugspannung des Gurts (12) ausübt,
**dadurch gekennzeichnet, dass**
der Gurt (12) als ein elastischer Gurt ausgeführt ist, und dass der Druckkörper (24) als Negativabdruck
der Rippenbogens ausgebildet ist und somit mit seiner Geometrie der Anatomie des menschlichen Rippenbogens angepasst ist und sich, in Gebrauch, in diesen einschmiegt.

2. Vorrichtung (10) nach Anspruch 1, wobei der elastische Gurt (12) geschlossen ist oder in Form eines zumindest teilweise elastischen Bandes ausgeführt ist, das im Bereich seiner Enden (14, 18) über Verbindungsmittel (16, 20) zur Einstellung seiner effektiven Länge um den Oberkörper des Patienten einstellbar miteinander verbindbar ist.

3. Vorrichtung (10) nach Anspruch 2, wobei die Verbindungsmittel (16, 20) in Form eines Klettverschluss ausgebildet sind, wobei vorzugsweise an einem Ende ein flächiger Klettflauschbereich (20) und an dem anderen Ende ein flächiger Kletthäkchenbereich (16) vorgesehen ist, oder wobei die Verbindungsmittel in Form wenigstens eines verstellbaren Verschlusselementes, wie beispielsweise wenigstens einer Gürtel-Schnallen-Verbindung oder eines Rastmechanismus oder dergleichen ausgebildet sind.

4. Vorrichtung (10) nach einem der vorangehenden Ansprüche, wobei der Druckkörper (24) in Form eines flächigen flexiblen Druckkörpers (24), insbesondere in Form eines Gummi-, Kunststoff- oder Silikonkörpers ausgebildet ist, der zum Angreifen unterhalb des Rippenbogens (R) geformt ist,
wobei vorzugsweise der Druckkörper (24) in Form eines abgerundeten Vielecks, insbesondere eines abgerundeten Dreiecks, ausgebildet ist,
wobei optional der Druckkörper (24) an seiner am Patienten angreifenden Oberfläche mit vorspringenden Noppen (28) versehen ist.

5. Vorrichtung nach Anspruch 4, wobei der Druckkörper mit einem umgekehrt V-förmigen Erhebungsbereich ausgebildet ist, der im Bereich des V-Scheitels seine größte Materialstärke aufweist.

6. Vorrichtung (10) nach einem Ansprüche 4 oder 5, wobei an der Rückseite des Druckkörpers (24) Befestigungsmittel vorgesehen sind, insbesondere ein Klettflauschbereich oder ein Kletthäkchenbereich.

7. Vorrichtung (10) nach einem der vorangehenden Ansprüche, wobei zwischen dem Druckkörper (24) und einer zur Anlage an dem Patienten vorgesehenen Innenseite (22) des Gurts (12) ein Druckkissen (34) vorgesehen ist,
wobei insbesondere das Druckkissen (34) aus einem formstabilen, vorzugsweise elastischen Material ausgebildet ist, vorzugsweise aus Gummi, Kunststoff oder Silikon ausgebildet ist.

8. Vorrichtung (10) nach Anspruch 7, wobei das Druckkissen (34) zumindest einseitig eine konvexe Form aufweist, wobei das Druckkissen (34) insbesondere im Querschnitt rechteckig, abgerundet rechteckig, elliptisch oder oval ausgebildet ist.

9. Vorrichtung (10) nach einem der Ansprüche 7 oder 8, wobei das Druckkissen (34) an seiner dem Gurt (12) zugewandten Seite sowie an seiner dem Druckkörper (24) zugewandten Seite jeweils mit Befestigungsmittel (36, 38), insbesondere mit einem Klettflauschbereich oder ein Kletthäkchenbereich ausgebildet ist.

10. Vorrichtung nach einem der Ansprüche 7 bis 9, wobei der Druckkörper mit einem Flauschmaterial überzogen ist, wobei das Flauschmaterial unmittelbar an dem Druckkörper angeformt ist oder in Form einer den Druckkörper aufnehmenden auswechselbaren Stofftasche ausgebildet ist.

11. Vorrichtung (10) nach einem der vorangehenden Ansprüche, wobei der Gurt (12) in einem Bereich, in dem der Druckkörper (24), gegebenenfalls unter Vermittlung des Druckkissens (34) nach einem der Ansprüche 6 bis 10, anbringbar ist, durch ein formstabiles Verstärkungselement (40) verstärkt ist,
wobei insbesondere der Bereich, in dem der Druckkörper (24), gegebenenfalls unter Vermittlung des Druckkissens (34) nach einem der Ansprüche 6 bis 8, anbringbar ist, in einem mittleren Abschnitt oder in einem Endbereich des Gurts (12) angeordnet ist.

12. Vorrichtung (10) nach Anspruch 11, wobei der Gurt (12) eine Aufnahmetasche (42) zum Aufnehmen des Verstärkungselements (40) aufweist.

13. Vorrichtung (10) nach einem der Ansprüche 11 oder 12, wobei das Verstärkungselement (40) von einer bogenförmig gekrümmten Verstärkungsleiste gebildet ist, die mit zwei gegenüber eine Mittelbereich (46) vorspringenden Endbereichen (48, 50) versehen ist, wobei das Verstärkungselement (50) derart in dem Gurt (12) aufgenommen oder an diesem angebracht ist, dass die beiden Endbereiche (48, 50), in Gebrauch, in Richtung zur Anlage an dem Patienten vorstehen.

14. Vorrichtung (10) nach Anspruch 13, wobei das Verstärkungselement (140) einen gerundet ausgebildeten Scheitelbereich (146) aufweist, der außermittig, vorzugsweise in einem Bereich eines Drittels der Gesamtlänge des Verstärkungselements (140), angeordnet ist, wobei vorzugsweise das Verstärkungselement (140) in seinem Scheitelbereich (146) einen Öffnungswinkel von 170° bis 120°, am meisten bevorzugt einen Öffnungswinkel von etwa 150° aufweist,
wobei insbesondere das Verstärkungselement (140) wahlweise in konvexer oder konkaver Anordnung relativ zu dem Druckkörper (124) oder/und wahlweise mit seinem Scheitelbereich (146) nahe oder entfernt von einer maximalen Materialstärke des Druckkörpers (124) anordenbar ist.

15. Vorrichtung (10) nach einem der vorangehenden Ansprüche, wobei in den Gurt (12) in einem mit der Haut des Patienten in Kontakt stehendem Bereich, insbesondere in dem Druckkörper (24) wenigstens ein Sensor zur Erfassung von Patientenparametern vorgesehen ist, insbesondere ein Sensor zum Erfassen von Puls, Herzfrequenz, Blutdruck, Blutzucker, Atemfrequenz, Körpertemperatur, Fettanteil, Wasseranteil, Muskelanteil, Body-Mass-Index oder dergleichen, wobei der Sensor über eine kabelgebundene oder kabellose Verbindung, beispielsweise über IR, Bluetooth, WI-FI oder eine Mobilfunkverbindung mit einem Endgerät zur Datenübermittlung koppelbar ist.

## Claims

1. A device (10) for the external application of a local compressive force below the costal arch (R) of a patient to reduce the stomach volume, comprising
- a belt (12) which can be placed around a patient's upper body in the region of the costal arch (R) under tensile stress, and
- a pressure body (24) which is attachable to the belt (12) and which is designed in such a way that, when in use, it exerts a compressive force on the stomach inside the patient in accordance with the tensile stress of the belt (12),
**characterized in that**
the belt (12) is designed as an elastic belt, and **in that**
the pressure body (24) is configured as a negative impression of the costal arch, thus adapting its geometry to the anatomy of the human costal arch and, when in use, nestling into the latter.

2. The device (10) of claim 1, wherein the elastic belt (12) is closed or designed as an at least partially elastic strap whose ends (14, 18) are adjustably connectable to one another through connecting means (16, 20) for adjusting its effective length around the patient's upper body.

3. The device (10) of claim 2, wherein the connecting means (16, 20) are configured as a hook-and-loop fastener, wherein preferably a planar fuzzy loop area (20) is provided at one end and a planar hook area (16) is provided at the other end, or wherein the connecting means are configured as at least one adjustable closure member, such as at least one belt-buckle connection or a latching mechanism or the like.

4. The device (10) of any one of the preceding claims, wherein the pressure body (24) is configured as a planar flexible pressure body (24), in particular as a rubber, plastic or silicone body, shaped to engage below the costal arch (R),
wherein the pressure body (24) is preferably configured as a rounded polygon, in particular a rounded triangle,
wherein the pressure body (24) is optionally provided with protruding nubs (28) on the surface engaging the patient.

5. The device of claim 4, wherein the pressure body is configured to have an inverted V-shaped projection area having its greatest material thickness in the area of the V apex.

6. The device (10) of claim 4 or 5, wherein fasteners, in particular a fuzzy loop area and a hook area, are provided on the back of the pressure body (24).

7. The device (10) of any one of the preceding claims, wherein a pressure pad (34) is provided between the pressure body (24) and an inner side (22) of the belt (12) intended for abutment against the patient,
wherein the pressure pad (34) in particular is made of an inherently stable, preferably elastic material, preferably made of rubber, plastic or silicone.

8. The device (10) of claim 7, wherein the pressure pad (34) has a convex shape on at least one side, wherein the pressure pad (34) has in particular a rectangular, a rounded rectangular, an elliptical or an oval cross-section.

9. The device (10) of claim 7 or 8, wherein the pressure pad (34) is configured with fasteners (36, 38), in particular with a fuzzy loop area or a hook area, on each of the side facing the belt (12) and the side facing the pressure body (24).

10. The device of any one of claims 7 to 9, wherein the pressure body is covered with a fuzzy material, the fuzzy material being molded directly onto the pressure body or formed as a replaceable fabric pocket that receives the pressure body.

11. The device (10) of any one of the preceding claims, wherein the belt (12) is reinforced by an inherently stable reinforcing member (40) in an area in which the pressure body (24) is attachable, optionally by means of the pressure pad (34) of any one of claims 6 to 10,
wherein in particular the area in which the pressure body (24) is attachable, optionally by means of the pressure pad (34) of any one of claims 6 to 8, is arranged in a central portion or in an end region of the belt (12).

12. The device (10) of claim 11, wherein the belt (12) includes a receiving pocket (42) for receiving the reinforcing member (40).

13. The device (10) of claim 11 or 12, wherein the reinforcing member (40) is formed by an arched reinforcing strip provided with two end regions (48, 50), each protruding relative to a central region (46), the reinforcing member (50) being received in or attached to the belt (12) such that, when in use, the two end regions (48, 50) project towards abutment against the patient.

14. The device (10) of claim 13, wherein the reinforcing member (140) has a rounded apex region (146) that is arranged off-center, preferably in a region of one third of the total length of the reinforcing member (140), wherein preferably the reinforcing member (140) has an opening angle ranging between 170° and 120° in its apex region (146), most preferably an opening angle of approximately 150°,
wherein the reinforcing member (140) in particular can be arranged optionally in a convex or concave arrangement relative to the pressure body (124) and/or optionally with its apex region (146) close to or away from a maximum material thickness of the pressure body (124).

15. The device (10) of any one of the preceding claims, wherein at least one sensor for detecting patient parameters, in particular a sensor for detecting the pulse, heart rate, blood pressure, blood sugar, respiratory rate, body temperature, fat percentage, water percentage, muscle percentage, body mass index or the like, is provided in the belt (12) in an area that is in contact with the patient's skin, in particular in the pressure body (24), wherein the sensor can be coupled to a terminal device for data transmission via a wired or wireless connection, for example via IR, Bluetooth, WI-FI or a mobile radio connection.

## Revendications

1. Dispositif (10) pour l'application externe d'une force de pression locale sous l'arc costal (R) d'un patient pour réduire le volume de l'estomac, comprenant :
- une ceinture (12) qui peut être placée sous tension autour du torse d'un patient dans la zone de l'arc costal (R), et
- un corps de pression (24) qui peut être fixé à la ceinture (12) et qui est conçu de telle sorte que, en utilisation, il exerce une force de pression sur l'estomac à l'intérieur du patient en fonction de la tension de la ceinture (12),
**caractérisé en ce que**
la ceinture (12) est réalisée sous la forme d'une ceinture élastique, et **en ce que**
le corps de pression (24) est réalisé sous la forme d'une empreinte négative de l'arc costal et est ainsi adapté par sa géométrie à l'anatomie de l'arc costal humain et, en utilisation, s'y adapte.

2. Dispositif (10) selon la revendication 1, dans lequel la ceinture élastique (12) est fermée ou réalisée sous la forme d'une bande au moins partiellement élastique qui, dans la zone de ses extrémités (14, 18), peut être reliée ensemble de manière réglable par des moyens de liaison (16, 20) pour régler sa longueur effective autour du torse du patient.

3. Dispositif (10) selon la revendication 2, dans lequel les moyens de liaison (16, 20) sont réalisés sous la forme d'une fermeture auto-agrippante, dans lequel il est prévu de préférence une zone plate de velours auto-agrippant (20) à une extrémité et une zone plate de crochets auto-agrippants (16) à l'autre extrémité, ou dans lequel les moyens de liaison sont réalisés sous la forme d'au moins un élément de fermeture réglable, comme par exemple au moins une liaison ceinture-boucle ou un mécanisme d'encliquetage ou analogue.

4. Dispositif (10) selon l'une des revendications précédentes, dans lequel le corps de pression (24) est réalisé sous la forme d'un corps de pression (24) plat et flexible, en particulier sous la forme d'un corps en caoutchouc, en matière plastique ou en silicone, qui est formé pour s'engager sous l'arc costal (R),
dans lequel, de préférence, le corps de pression (24) est réalisé sous la forme d'un polygone arrondi, en particulier d'un triangle arrondi,
dans lequel, en option, le corps de pression (24) est pourvu de picots (28) en saillie sur sa surface s'engageant sur le patient.

5. Dispositif selon la revendication 4, dans lequel le corps de pression est réalisé avec une zone en relief en forme de V inversé, qui a sa plus grande épaisseur de matériau dans la zone du sommet du V.

6. Dispositif (10) selon l'une des revendications 4 ou 5, dans lequel des moyens de fixation sont prévus sur la face arrière du corps de pression (24), en particulier une zone de velours auto-agrippant ou une zone de crochets auto-agrippants.

7. Dispositif (10) selon l'une des revendications précédentes, dans lequel un coussin de pression (34) est prévu entre le corps de pression (24) et une face intérieure (22) de la ceinture (12) prévue pour s'appliquer contre le patient,
dans lequel le coussin de pression (34) est en particulier réalisé dans un matériau de forme stable, de préférence élastique, et est de préférence réalisé en caoutchouc, en matière plastique ou en silicone.

8. Dispositif (10) selon la revendication 7, dans lequel le coussin de pression (34) présente au moins d'un côté une forme convexe, dans lequel le coussin de pression (34) est en particulier réalisé avec une section transversale rectangulaire, rectangulaire arrondie, elliptique ou ovale.

9. Dispositif (10) selon l'une des revendications 7 ou 8, dans lequel le coussin de pression (34) est réalisé avec des moyens de fixation (36, 38) sur sa face tournée vers la ceinture (12) ainsi que sur sa face tournée vers le corps de pression (24), en particulier avec une zone de velours auto-agrippant ou une zone de crochets auto-agrippants.

10. Dispositif selon l'une des revendications 7 à 9, dans lequel le corps de pression est recouvert d'un matériau pelucheux, le matériau pelucheux étant directement formé sur le corps de pression ou étant réalisé sous la forme d'une poche en tissu interchangeable recevant le corps de pression.

11. Dispositif (10) selon l'une des revendications précédentes, dans lequel la ceinture (12) est renforcée par un élément de renfort de forme stable (40) dans une zone dans laquelle le corps de pression (24) peut être appliqué, le cas échéant par l'intermédiaire du coussin de pression (34) selon l'une des revendications 6 à 10,
dans lequel, en particulier, la zone dans laquelle le corps de pression (24) peut être appliqué, le cas échéant par l'intermédiaire du coussin de pression (34) selon l'une des revendications 6 à 8, est disposée dans une partie centrale ou dans une zone d'extrémité de la ceinture (12).

12. Dispositif (10) selon la revendication 11, dans lequel la ceinture (12) comprend une poche de réception (42) destinée à recevoir l'élément de renfort (40).

13. Dispositif (10) selon l'une des revendications 11 ou 12, dans lequel l'élément de renfort (40) est formé par une lame de renfort incurvée en arc de cercle qui est pourvue de deux zones d'extrémité (48, 50) en saillie par rapport à une zone centrale (46), dans lequel l'élément de renfort (50) est reçu dans la ceinture (12) ou fixé à celle-ci de telle sorte que les deux zones d'extrémité (48, 50), en utilisation, fassent saillie dans la direction d'application sur le patient.

14. Dispositif (10) selon la revendication 13, dans lequel l'élément de renfort (140) comprend une zone de sommet (146) de forme arrondie qui est disposée de manière excentrée, de préférence dans une zone d'un tiers de la longueur totale de l'élément de renfort (140), dans lequel, de préférence, l'élément de renfort (140) présente dans sa zone de sommet (146) un angle d'ouverture de 170° à 120°, de manière la plus préférée un angle d'ouverture d'environ 150°,
dans lequel, en particulier, l'élément de renfort (140) peut être disposé au choix dans une disposition convexe ou concave par rapport au corps de pression (124) ou/et au choix avec sa zone de sommet (146) proche ou éloignée d'une épaisseur de matériau maximale du corps de pression (124).

15. Dispositif (10) selon l'une des revendications précédentes, dans lequel il est prévu dans la ceinture (12), dans une zone en contact avec la peau du patient, en particulier dans le corps de pression (24), au moins un capteur pour détecter des paramètres du patient, en particulier un capteur pour détecter le pouls, la fréquence cardiaque, la pression sanguine, la glycémie, la fréquence respiratoire, la température corporelle, le pourcentage de graisse, le pourcentage d'eau, le pourcentage de muscle, l'indice de masse corporelle ou analogue, dans lequel le capteur peut être couplé à un terminal pour la transmission de données par une liaison filaire ou sans fil, par exemple par IR, Bluetooth, WI-FI ou une liaison de téléphonie mobile.
